(19) Europäisches Patentamt
European Patent Office
Office européen des brevets

(11) Veröffentlichungsnummer: **0 104 300**
**B1**

(12) # EUROPÄISCHE PATENTSCHRIFT

(45) Veröffentlichungstag der Patentschrift: **26.03.86**

(51) Int. Cl.⁴: **C 07 D249/08**, A 61 K 31/41

(21) Anmeldenummer: **83100014.6**

(22) Anmeldetag: **04.01.83**

(54) **Diazolyl-alkanole, Verfahren zu ihrer Herstellung und ihre Verwendung als antimykotische Mittel.**

(30) Priorität: **03.08.82 DE 3228870**

(43) Veröffentlichungstag der Anmeldung: **04.04.84 Patentblatt 84/14**

(45) Bekanntmachung des Hinweises auf die Patenterteilung: **26.03.86 Patentblatt 86/13**

(84) Benannte Vertragsstaaten: **AT BE CH DE FR GB IT LI LU NL SE**

(56) Entgegenhaltungen:
**EP-A- 0 022 969**
**EP-A- 0 043 419**
**DE-A- 2 820 489**
**US-A- 3 991 200**

(73) Patentinhaber: **BAYER AG**
**Konzernverwaltung RP Patentabteilung**
**D-5090 Leverkusen 1 Bayerwerk (DE)**

(72) Erfinder: **Regel, Erik, Dipl.-Ing.**
**Untere Bergerheide 26**
**D-5600 Wuppertal 1 (DE)**
Erfinder: **Büchel, Karl Heinz, Prof. Dr.**
**Dabringhausener Strasse 42**
**D-5093 Burscheid (DE)**
Erfinder: **Plempel, Manfred, Dr.**
**Zwengenberger Strasse 3c**
**D-5657 Haan (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 104 300 B1

**Beschreibung**

Die Erfindung betrifft neue Diazolyl-alkanole, ein Verfahren zu ihrer Herstellung sowie ihre Verwendung als Antimykotika.

Es ist bereits bekannt geworden, daß Hydroxypropylimidazole, wie beispielsweise 2-(4-Biphenylyl)-1-(2,4-dichlorphenyl)-3-(imidazol-1-yl)-2-propanol, bzw. 1-Hydroxyethyl-azol-Derivate, wie beispielsweise 2-(4-Chlorphenoxymethyl)-3,3-dimethyl-1-(imidazol-1-yl)-butan-2-ol oder 2-(2-Methylphenoxymethyl)-3,3-dimethyl-1-(1,2,4-triazol-1-yl)- und (Imidazol-1-yl)-butan-2-ol, gute antimykotische Eigenschaften aufweisen (vergleiche DE-OS 28 20 489 bzw. DE-OS 30 18 865). Die Wirkung dieser Verbindungen ist jedoch, insbesondere in-vivo, nicht immer voll befriedigend.

Es wurden neue Diazolyl-alkanole der allgemeinen Formel I

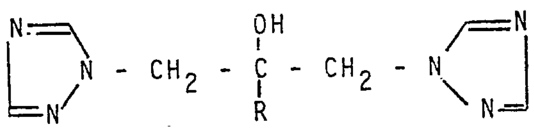

(I)

in welcher R für Alkenyl und Alkinyl mit jeweils 2 bis 6 Kohlenstoffatomen sowie für jeweils gegebenenfalls einfach bis dreifach, gleich oder verschieden substituiertes Phenylalkinyl mit 2 bis 4 Kohlenstoffatomen im Alkinylteil, und Phenylalkenyl mit 2 bis 4 Kohlenstoffatomen im Alkenylteil, wobei jeweils als Phenylsubstituenten genannt seien : Halogen, Alkyl mit 1 bis 4 Kohlenstoffatomen, Alkoxy und Alkylthio mit jeweils 1 bis 2 Kohlenstoffatomen, Alkoxyalkyl mit 1 bis 2 Kohlenstoffatomen in jedem Alkylteil sowie gegebenenfalls durch Halogen substituiertes Phenyl, und deren physiologisch verträglichen Säureadditions-Salze gefunden.

Weiterhin wurde gefunden, daß man die Diazolyl-alkanole der Formel I erhält, wenn man Dihalogenalkanole der allgemeinen Formel II

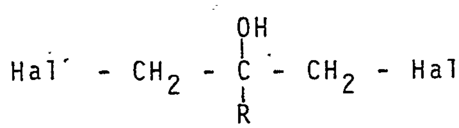

(II)

in welcher

R die oben angegebene Bedeutung hat und

Hal für Halogen steht,

mit 1,2,4-Triazolen der allgemeinen Formel III

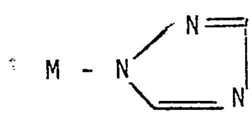

(III)

in welcher M für Wasserstoff oder ein Alkalimetall steht, in Gegenwart eines Verdünnungsmittels und gegebenenfalls in Gegenwart eines Säurebindemitells umsetzt.

An die so erhaltenen Verbindungen der allgemeinen Formel I kann gegebenenfalls anschließend ein Säure addiert werden.

Die neuen Diazolyl-alkanole der allgemeinen Formel I weisen starke antimykotische Eigenschaften auf. Dabei zeigen überraschenderweise die erfindungsgemäßen Verbindungen eine bessere, therapeutisch nutzbare in-vivo-Wirksamkeit als die aus dem Stand der Technik bekannten Verbindungen 2-(4-Biphenyl-yl)-1-(2,4-dichlorphenyl)-3-(imidazol-1-yl)-2-propanol ; 2-(4-Chlorphenoxy-methyl)-3,3-dimethyl-1-(imidazol-1-yl)-butan-2-ol und 2-(2-Methylphenoxymethyl)-3,3-dimethyl-1-(1,2,4-triazol-1-yl)- bzw. (imidazol-1-yl)-butan-2-ol, welches chemisch naheliegende Verbindungen sind. Die erfindungsgemäßen Stoffe stellen somit eine Bereicherung der Pharmazie dar.

Außerdem sind die neuen Diazolyl-alkanole interessante Zwischenprodukte. So können z. B. die Verbindungen der allgemeinen Formel I an der Hydroxy-Gruppe in üblicher Weise in die entsprechenden Ether übergeführt werden. Weiterhin können durch Umsetzung mit z. B. Acetylhalogeniden oder Carbamoylhalogeniden in prinzipiell bekannter Weise Acyl- oder Carbamoyl-Derivate der Verbindungen der allgemeinen Formel I erhalten werden.

Verwendet man beispielsweise 4-Chlor-3-chlormethyl-3-hydroxy-1-phenyl-1-butin und 1,2,4-Triazol als Ausgangsstoffe sowie Kaliumcarbonat als Säurebindemittel, so kann der Reaktionsablauf des erfindungsgemäßen Verfahrens durch das folgende Formelschema wiedergegeben werden :

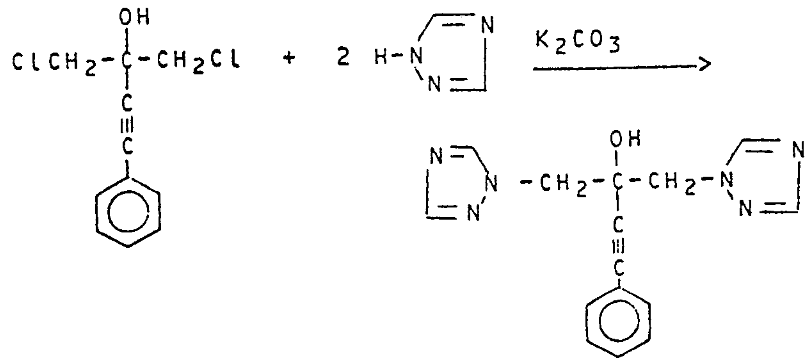


Die für das erfindungsgemäße Verfahren als Ausgangsstoffe zu verwendenden Dihalogenalkanole sind durch die allgemeine Formel II allgemein definiert. In dieser Formel steht R vorzugsweise für diejenigen Reste, die bereits im Zusammenhang mit der Beschreibung der erfindungsgemäßen Stoffe der allgemeinen Formel I vorzugsweise für diesen Substituenten genannt wurden.-

Hal steht vorzugsweise für Chlor.

Die Dihalogenalkanole der allgemeinen Formel II sind noch nicht bekannt ; sie können in allgemein bekannter Art und Weise erhalten werden, indem man 1,3-Dihalogenaceton, wie insbesondere 1,3-Dichloraceton mit einem entsprechenden Grignardreagenz umsetzt [vergleiche hierzu auch J. Org. Chem. *27,* 2242 (1961) sowie die Herstellungsbeispiele].

Die außerdem für das erfindungsgemäße Verfahren als Ausgangsstoffe zu verwendenden 1,2,4-Triazole sind durch die allgemeine Formel III allgemein definiert. In dieser Formel steht M vorzugsweise für Wasserstoff, Natrium oder Kalium.

Die 1,2,4-Triazole der allgemeinen Formel III sind allgemein bekannte Verbindungen der organischen Chemie. Die Alkalisalze werden durch Umsetzung von 1,2,4-Triazol mit Natrium- oder Kaliumethylat oder durch Umsetzung von 1,2,4-Triazol mit der äquivalenten Menge entsprechenden Alkalihydrids erhalten.

Als Verdünnungsmittel kommen für das erfindungsgemäße Verfahren vorzugsweise inerte organische Lösungsmittel in Frage. Hierzu gehören vorzugsweise Ketone, wie insbesondere Aceton und Methylethylketon ; Nitrile, wie insbesondere Acetonitril ; Alkohole wie insbesondere Ethanol und Isopropanol ; Ether, wie insbesondere Tetrahydrofuran oder Dioxan ; Formamide, wie insbesondere Dimethylformamid ; sowie aromatische und halogenierte Kohlenwasserstoffe.

Das erfindungsgemäße Verfahren wird gegebenenfalls in Gegenwart eines Säurebindemittels vorgenommen. Man kann alle üblicherweise verwendbaren anorganischen oder organischen Säurebinder Zugeben, wie Alkalicarbonate, beispielsweise Natrium- und Kaliumcarbonat ; oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicycloöctan sowie auch ein entsprechender überschuß an 1,2,4-Triazol.

Die Reaktionstemperaturen können beim erfindungsgemäßen Verfahren in einem größeren Bereich variiert werden. Im allgemeinen arbeitet man zwischen etwa 20 bis etwa 150 °C, vorzugsweise bei 20 bis 120 °C.

Bei der Durchführung des erfindungsgemäßen Verfahrens setzt man auf 1 Mol der Dihalogenalkanole der allgemeinen Formel II 2 bis 4 Mol 1,2,4-Triazol der allgemeinen Formel III und gegebenenfalls 2 bis 4 Mol Säurebinder ein. Die Isolierung der Verbindungen der allgemeinen Formel I erfolgt in üblicher Art und Weise.

Zur Herstellung von Säureadditions-Salzen der Verbindungen der allgemeinen Formel I kommen alle physiologisch verträglichen Salze in Frage. Hierzu gehören vorzugsweise die Halogenwasserstoffsäuren, wie z. B. die Chlorwasserstoff und Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure, Milchsäure sowie Sulfonsäuren, wie z. B. p-Toluolsulfonsäure und 1,5-Naphthalindisulfonsäure.

Die Salze der Verbindungen der allgemeinen Formel I können in einfacher Weise nach üblichen Salzbildungsmethoden, z. B. durch Lösen einer Verbindung der allgemeinen Formel I in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Verbindungen der allgemeinen Formel I und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie

Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden :

Dermatomykosen und Systemmykosen, durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden :

Alle Dermatomykosen und Systemmykosen, insbesondere solche, die durch die obengenannten Erreger hervorgerufen werden.

Zur Erfindung gehören auch pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur Erfindung gehören schließlich pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal,vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

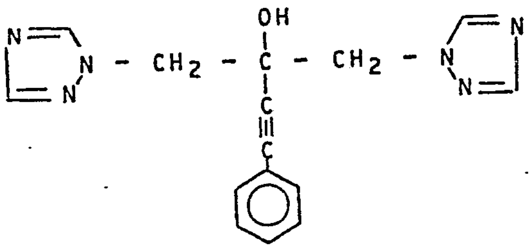

Zu einem Gemisch von 27,6 g (0,4 Mol) 1,2,4-Triazol und 55,2 g (0,4 Mol) Kaliumcarbonat in 300 ml Aceton werden 22,9 g (0,1 Mol) 4-Chlor-3-chlormethyl-3-hydroxy-1-phenyl-1-butin getropft. Man läßt das Reaktionsgemisch 12 Stunden unter Rückfluß rühren. Nach dem Abkühlen wird filtriert. Das Filtrat wird im Vakuum eingeengt und der ölige Rückstand chromatographisch gereinigt (Kieselgel 60 Merck, Chloroform). Man erhält 8 g (27 % der Theorie) 3-Hydroxy-1-phenyl-4-(1,2,4-triazol-1-yl)-3-(1,2,4-triazol-1-yl-methyl)-1-butin vom Schmelzpunkt 140 °C.

### Herstellung des Ausgangsproduktes

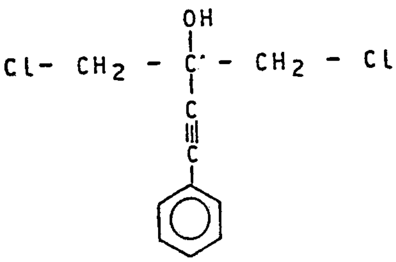

Eine Suspension von Phenylacetylenyl-magnesiumbromid in Diethylether — hergestellt aus 12 g (0,5 Mol) Magnesium, 54,5 g (0,5 Mol) Ethylbromid und 51 g (0,5 Mol) Phenylacetylen in 500 ml Diethylether — wird bei − 60 °C in eine Lösung von 63,5 g (0,5 Mol) 1,3-Dichloraceton in 300 ml Diethylether getropft. Nach 2 Stunden läßt man das Reaktionsgemisch auf 0 °C erwärmen und versetzt mit 49,3 g (0,82 Mol) Essigsäure, wobei weitere Erwärmung auf ca. 20 °C erfolgt. Nach Zugabe von 200 ml Wasser wird die Etherphase abgetrennt, mit Wasser gewaschen, über Natriumsulfat getrocknet und im Vakuum eingeengt. Man erhält 114 g (99 % der Theorie) 4-Chlor-3-chlormethyl-3-hydroxy-1-phenyl-1-butin vom Brechungsindex $n_D^{20}$ = 1,557 5.

In entsprechender Weise und gemäß dem erfindungsgemäßen Verfahren werden die folgenden Verbindungen der allgemeinen Formel I

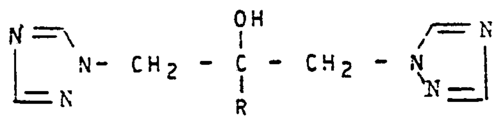

(I)

erhalten :

Tabelle 1

| Bsp. Nr. | R | Schmelzpunkt(°C) bzw. $n_D^{20}$ |
|---|---|---|
| 2 | 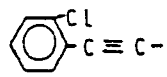 | 126 |
| 3 | 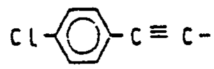 | 164 |
| 4 | 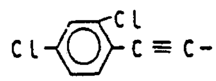 | 128 |
| 5 | 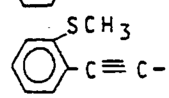 | 1,5985 |
| 6 | 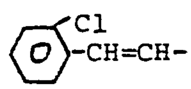 | 65 |

Entsprechend Beispiel 1 werden die folgenden Zwischenprodukte der allgemeinen Formel (IIa)

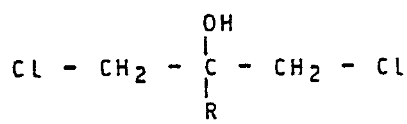

(IIa)

erhalten :

Tabelle 2

| Bsp. Nr. | R | Physikalische Konstante |
|---|---|---|
| II-2 | 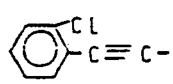 | zähes Öl |
| II-3 | 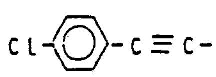 | $n_D^{20}$=1,5744 |
| II-4 | 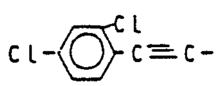 | $n_D^{20}$=1,5818 |
| II-5 | 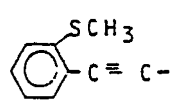 | zähes Öl |

Verwendungsbeispiele

In dem nachfolgenden Beispiel werden die nachstehend angegebenen Verbindungen als Vergleichsversuche eingesetzt :

(A) 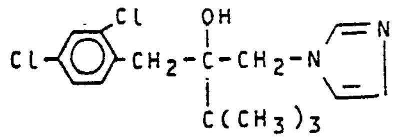

(B) 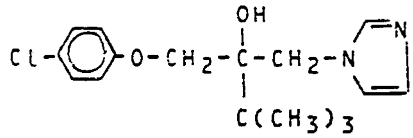

(C) 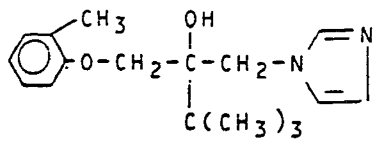

(D) 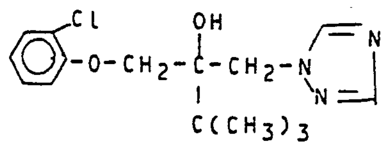

Beispiel A

Antimykotische in-vivo-Wirksamkeit (oral) bei Mäusecandidose

Versuchsbeschreibung :

Mäuse vom Typ SPF-$CF_1$ wurden intravenös mit 1-2 × $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 50-100 mg/kg Körpergewicht der Präparate oral behandelt.

Ergebnis :

Unbehandelte Tiere starben 3 bis 6 Tage post infektionem. Die Überlebensrate am 6. Tag post infektionem betrug bei unbehandelten Kontrolltieren etwa 5 %.

In diesem Test zeigten z. B. die erfindungsgemäßen Verbindungen eine bessere Wirkung als die aus dem Stand der Technik bekannten Verbindungen 1 und 3.

Zeichenerklärung :

+++++ = sehr gute Wirkung = 90 % Überlebende am 6. Tag p.i.
++++ = gute Wirkung = 80 % Überlebende am 6. Tag p.i.
+++ = Wirkung = 60 % Überlebende am. 6. Tag p.i.
++ = schwache Wirkung = 40 % Überlebende am. 6. Tag p.i.
+ = Spur Wirkung =
k.W. = keine Wirkung

Tabelle A

Antimykotische in-vivo-Wirksamkeit (oral) bei Mäusecandidose

| Wirkstoff | Wirkung |
|---|---|
| (A) (bekannt) | k.W. |
| (B) (bekannt) | k.W. |
| (C) (bekannt) | k.W. |
| (D) (bekannt) | k.W. |

Verbindungen gemäß Herstellungsbeispiel

| | |
|---|---|
| 1 | +++ |
| 3 | +++++ |

Beispiel B/Formulierungen

1) Lösung :

| | |
|---|---|
| Wirkstoff gemäß allgemeiner Formel I : | 10 g |
| Alkohol, rein (96 %-ig) : | 300 g |
| Isopropylmyristat : | 526 g |
| | 836 g |

2) Creme :

| | |
|---|---|
| Wirkstoff gemäß allgemeiner Formel I : | 10 g |
| Arlacel 60 (Sorbitan-monostearat) : | 20 g |
| Tween 60 (Polyoxyethylen(20)-sorbitanmonostearat) : | 15 g |
| Walrat, künstlich (Mischung von Estern von gesättigten Fettsäuren $C_{14}$-$C_{18}$ und Fettalkoholen $C_{14}$-$C_{18}$) : | 30 g |
| Lanette O (Gemisch aus Cetylalkohol und Stearylalkohol) : | 100 g |
| Entanol G (2-Octyl-dodecanol) : | 135 g |
| Benzylalkohol : | 10 g |
| Wasser, entmineralisiert : | 680 g |
| | 1 000 g |

**Patentansprüche**

1. Diazolyl-alkanole der allgemeinen Formel I

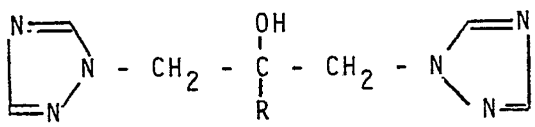

(I)

in welcher R für Alkenyl und Alkinyl mit jeweils 2 bis 6 Kohlenstoffatomen, für jeweils gegebenenfalls einfach bis dreifach, gleich oder verschieden substituiertes Phenylalkenyl mit 2 bis 4 Kohlenstoffatomen im Alkenylteil oder Phenylalkinyl mit 2 bis 4 Kohlenstoffatomen im Alkinylteil steht, wobei jeweils als Phenylsubstituenten genannt seien : Halogen, Alkyl mit 1 bis 4 Kohlenstoffatomen, Alkoxy und Alkylthio mit jeweils 1 bis 2 Kohlenstoffatomen, Alkoxyalkyl mit 1 bis 2 Kohlenstoffatomen, Alkoxyalkyl mit 1 bis 2 Kohlenstoffatomen in jedem Alkylteil sowie gegebenenfalls durch Halogen substituiertes Phenyl und deren physiologisch verträglichen Säureadditions-Salze.

2. 3-Hydroxy-1-(p-chlorphenyl)-4-(1,2,4-triazol-1-yl)-3-(1,2,4-triazol-1-yl-methyl)-1-butin.

3. Verfahren zur Herstellung von Diazolyl-alkanolen der allgemeinen Formel I in Anspruch 1, dadurch gekennzeichnet, daß man Dihalogenalkanole der allgemeinen Formel II

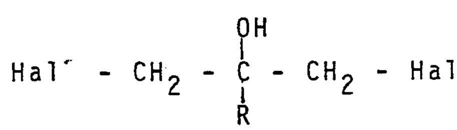

(II)

in welcher
R die oben angegebene Bedeutung hat und
Hal für Halogen steht,
mit 1,2,4-Triazolen der allgemeinen Formel III

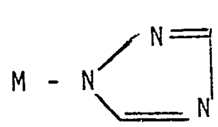

(III)

in welcher M für Wasserstoff oder ein Alkalimetall steht, in Gegenwart eines Verdünnungsmittels und gegebenenfalls in Gegenwart eines Säurebindemittels umsetzt.

4. Arzneimittel gekennzeichnet durch einen Gehalt an mindestens einem Diazolyl-alkanol gemäß Anspruch 1.

**Claims**

1. Diazolylalkanols of the general formula I

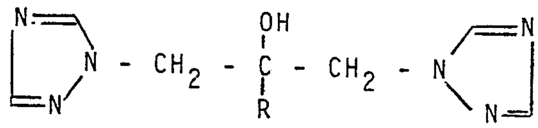

(I)

in which R represents alkenyl and alkynyl, each having 2 to 6 carbon atoms, and represents phenylalkenyl, having 2 to 4 carbon atoms in the alkenyl part, or phenylalkynyl, having 2 to 4 carbon atoms in the alkynyl part, each optionally identically or differently substituted once to three times, the phenyl substituents which may be mentioned in each case being : halogen, alkyl having 1 to 4 carbon atoms, alkoxy and alkylthio each having 1 to 2 carbon atoms, alkoxyalkyl having 1 to 2 carbon atoms in each alkyl part and phenyl which is optionally substituted by halogen and physiologically tolerated acid addition salts thereof.

2. 3-Hydroxy-1-(p-chlorophenyl)-4-(1,2,4-triazol-1-yl)-3-(1,2,4-triazol-1-yl-methyl)-1-butyne.

3. Process for the preparation of diazolylalkanols of the general formula I in Claim 1, characterised in that dihalogenoalkanols of the general formula II

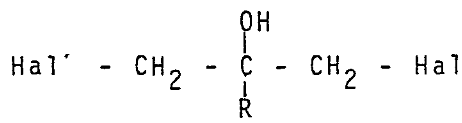

(II)

in which
R has the abovementioned meaning and
Hal represents halogen,
are reacted with 1,2,4-triazoles of the general formula III

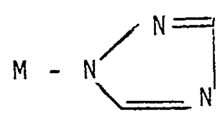

(III)

in which M represents hydrogen or an alkali metal, in the presence of a diluent and optionally in the presence of an acid-binding agent.

4. Medicaments characterised by a content of at least one diazolylalkanol according to Claim 1.

**Revendications**

1. Diazolylalcanols de formule générale I

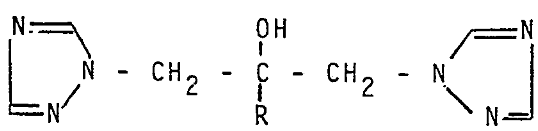

(I)

dans laquelle R est un groupe alcényle et alcynyle ayant dans chaque cas 2 à 6 atomes de carbone, un groupe phénylalcényle ayant 2 à 4 atomes de carbone dans la partie alcénylique ou un groupe phénylalcynyle ayant 2 à 4 atomes de carbone dans la partie alcynylique, portant chacun éventuellement 1 à 3 substituants égaux ou différents, et on mentionne alors comme substituants de chacun des groupes phényle : un halogène, un groupe alkyle ayant 1 à 4 atomes de carbone, un groupe alkoxy et un groupe alkylthio ayant chacun 1 ou 2 atomes de carbone, un groupe alkoxyalkyle ayant 1 ou 2 atomes de carbone, un groupe alkoxyalkyle ayant 1 ou 2 atomes de carbone dans chaque partie alkyle, ainsi qu'un groupe phényle éventuellement substitué par un halogène et leurs sels d'addition d'acides physiologiquement acceptables.

2. Le 3-hydroxy-1-(p-chlorophényl)-4-(1,2,4-triazole-1-yl)-3-(1,2,4-triazole-1-yl-méthyl)-1-butyne.

3. Procédé de production de diazolylalcanols de formule générale I suivant la revendication 1, caractérisé en ce qu'on fait réagir des dihalogénalcanols de formule générale II

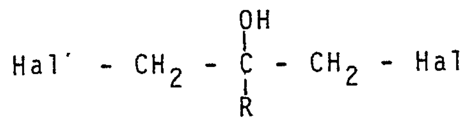

(II)

dans laquelle

R a la définition indiquée ci-dessus, et

Hal représente un halogène,

avec des 1,2,4-triazoles de formule générale III

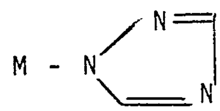

(III)

dans laquelle M représente l'hydrogène ou un métal alcalin en présence d'un diluant et le cas échéant en présence d'un accepteur d'acide.

4. Médicaments caractérisés par une teneur en au moins un diazolylalcanol suivant la revendication 1.